# EUROPÄISCHE PATENTSCHRIFT

(11) **EP 1 425 392 B1**
(45) Veröffentlichungstag und Bekanntmachung des Hinweises auf die Patenterteilung: **23.11.2005**
(21) Anmeldenummer: 02767395.3
(22) Anmeldetag: 16.08.2002
(51) Int. Cl.: C12N 9/88

(54) **PYRUVATDECARBOXYLASE-MUTANTE, DEREN HERSTELLUNG UND VERWENDUNG**
NOVEL PYRUVATE DECARBOXYLASE, PRODUCTION AND USE THEREOF
NOUVELLE PYRUVATE DECARBOXYLASE, SA PRODUCTION ET SON UTILISATION

(30) Priorität: 31.08.2001 DE 10142467
(43) Veröffentlichungstag der Anmeldung: 09.06.2004
(73) Patentinhaber: BASF Aktiengesellschaft, 67056 Ludwigshafen (DE)
(72) Erfinder: BREUER, Michael, 67117 Limburgerhof (DE); HAUER, Bernhard, 67136 Fussgönheim (DE); FRIEDRICH, Thomas, 64283 Darmstadt (DE)
(86) Internationale Anmeldenummer: PCT/EP2002/009159
(87) Internationale Veröffentlichungsnummer: WO 2003/020921

(56) Entgegenhaltungen:
- WO-A-96/37620
- WO-A-99/09195
- IWAN P. ET AL., : "studies on the continous production of (r)-(-) phenylacetylcarbinol in an enzyme-membrane reactor" J. MOLECULAR CATALYSIS B: ENZYMATIC , Bd. 11, 22. Januar 2001 (2001-01-22), Seiten 387-396, XP002249485
- BRUHN H ET AL: "THE REPLACEMENT OF TRP392 BY ALANINE INFLUENCES THE DECARBOXYLASE/ CARBOLIGASE ACTIVITY AND STABILITY OF PYRUVATE DECARBOXYLASE FROM ZYMOMONAS MOBILIS" EUROPEAN JOURNAL OF BIOCHEMISTRY, BERLIN, DE, Bd. 234, Nr. 2, 1. Dezember 1995 (1995-12-01), Seiten 650-655, XP000607025 ISSN: 0014-2956
- CANDY J. & DUGGLEBY R.: "Structure and properties of pyruvate decarboxylase and site-directed mutagenesis of the Zymomonas mobilis enzyme" BIOCHIMICA BIOPHYSICA ACTA, Bd. 1385, 1998, Seiten 232-338, XP004278419

### SEQUENCE LISTING

<110> BASF Aktiengesellschaft
<120> Novel Pyruvate Decarboxylase
<130> PF52815
<140> PCT/EP02/09159
   <141> 2002-08-16
<160> 2
<170> PatentIn version 3.1
<210> 1
   <211> 1707
   <212> DNA
   <213> Zymomonas mobilis
<220>
   <221> CDS
   <222> (1)..(1707)
   <223>
<400> 1
   atg agt tat act gtc ggt acc tat tta gcg gag cgg ctt gtc cag att         48
   Met Ser Tyr Thr Val Gly Thr Tyr Leu Ala Glu Arg Leu Val Gln Ile
   1               5                   10                  15
   ggt ctc aag cat cac ttc gca gtc gcg ggc gac tac aac ctc gtc ctt         96
   Gly Leu Lys His His Phe Ala Val Ala Gly Asp Tyr Asn Leu Val Leu
               20                  25                  30
   ctt gac aac ctg ctt ttg aac aaa aac atg gag cag gtt tat tgc tgt        144
   Leu Asp Asn Leu Leu Leu Asn Lys Asn Met Glu Gln Val Tyr Cys Cys
           35                  40                  45
   aac gaa ctg aac tgc ggt ttc agt gca gaa ggt tat gct cgt gcc aaa        192
   Asn Glu Leu Asn Cys Gly Phe Ser Ala Glu Gly Tyr Ala Arg Ala Lys
       50                  55                  60
   ggc gca gca gca gcc gtc gtt acc tac agc gtt ggt gcg ctt tcc gca        240
   Gly Ala Ala Ala Ala Val Val Thr Tyr Ser Val Gly Ala Leu Ser Ala
   65                  70                  75                  80
   ttt gat gct atc ggt ggc gcc tat gca gaa aac ctt ccg gtt atc ctg      288
   Phe Asp Ala Ile Gly Gly Ala Tyr Ala Glu Asn Leu Pro Val Ile Leu
                   85                  90                  95
   atc tcc ggt gct ccg aac aac aac gac cac gct gct ggt cat gtg ttg      336
   Ile Ser Gly Ala Pro Asn Asn Asn Asp His Ala Ala Gly His Val Leu
               100                 105                 110
   cat cac gct ctt ggc aaa acc gac tat cac tat cag ttg gaa atg gcc      384
   His His Ala Leu Gly Lys Thr Asp Tyr His Tyr Gln Leu Glu Met Ala
           115                 120                 125
   aag aac atc acg gcc gcc gct gaa gcg att tac acc ccg gaa gaa gct      432
   Lys Asn Ile Thr Ala Ala Ala Glu Ala Ile Tyr Thr Pro Glu Glu Ala
       130                 135                 140
   ccg gct aaa atc gat cac gtg atc aaa act gct ctt cgc gag aag aag      480
   Pro Ala Lys Ile Asp His Val Ile Lys Thr Ala Leu Arg Glu Lys Lys
   145                 150                 155                 160
   ccg gtt tat ctc gaa atc gct tgc aac att gct tcc atg ccc tgc gcc      528
   Pro Val Tyr Leu Glu Ile Ala Cys Asn Ile Ala Ser Met Pro Cys Ala
                   165                 170                 175
   gct cct gga ccg gca agt gca ttg ttc aat gac gaa gcc agc gac gaa      576
   Ala Pro Gly Pro Ala Ser Ala Leu Phe Asn Asp Glu Ala Ser Asp Glu
               180                 185                 190
   gca tcc ttg aat gca gcg gtt gac gaa acc ctg aaa ttc atc gcc aac      624
   Ala Ser Leu Asn Ala Ala Val Asp Glu Thr Leu Lys Phe Ile Ala Asn
           195                 200                 205
   cgc gac aaa gtt gcc gtc ctc gtc ggc agc aag ctg cgc gct gct ggt      672
   Arg Asp Lys Val Ala Val Leu Val Gly Ser Lys Leu Arg Ala Ala Gly
       210                 215                 220
   gct gaa gaa gct gct gtt aaa ttc acc gac gct ttg ggc ggt gca gtg      720
   Ala Glu Glu Ala Ala Val Lys Phe Thr Asp Ala Leu Gly Gly Ala Val
   225                 230                 235                 240
   gct act atg gct gct gcc aag agc ttc ttc cca gaa gaa aat gcc aat      768
   Ala Thr Met Ala Ala Ala Lys Ser Phe Phe Pro Glu Glu Asn Ala Asn
                   245                 250                 255
   tac att ggt acc tca tgg ggc gaa gtc agc tat ccg ggc gtt gaa aag      816
   Tyr Ile Gly Thr Ser Trp Gly Glu Val Ser Tyr Pro Gly Val Glu Lys
               260                 265                 270
   acg atg aaa gaa gcc gat gcg gtt atc gct ctg gct cct gtc ttc aac      864
   Thr Met Lys Glu Ala Asp Ala Val Ile Ala Leu Ala Pro Val Phe Asn
           275                 280                 285
   gac tac tcc acc act ggt tgg acg gat atc cct gat cct aag aaa ctg      912
   Asp Tyr Ser Thr Thr Gly Trp Thr Asp Ile Pro Asp Pro Lys Lys Leu
       290                 295                 300
   gtt ctc gct gaa ccg cgt tct gtc gtt gtc aac ggc att cgc ttc ccc      960
   Val Leu Ala Glu Pro Arg Ser Val Val Val Asn Gly Ile Arg Phe Pro
   305                 310                 315                 320
   agc gtt cat ctg aaa gac tat ctg acc cgt ttg gct cag aaa gtt tcc     1008
   Ser Val His Leu Lys Asp Tyr Leu Thr Arg Leu Ala Gln Lys Val Ser
                   325                 330                 335
   aag aaa acc ggt tct ttg gac ttc ttc aaa tcc ctc aat gca ggt gaa     1056
   Lys Lys Thr Gly Ser Leu Asp Phe Phe Lys Ser Leu Asn Ala Gly Glu
               340                 345                 350
   ctg aag aaa gcc gct ccg gct gat ccg agt gct ccg ttg gtc aac gca      1104
   Leu Lys Lys Ala Ala Pro Ala Asp Pro Ser Ala Pro Leu Val Asn Ala
           355                 360                 365
   gaa atc gcc cgt cag gtc gaa gct ctt ctg acc ccg aac acg acg gtt      1152
   Glu Ile Ala Arg Gln Val Glu Ala Leu Leu Thr Pro Asn Thr Thr Val
       370                 375                 380
   att gct gaa acc ggt gac tct tgg ttc aat gct cag cgc atg aag ctc      1200
   Ile Ala Glu Thr Gly Asp Ser Trp Phe Asn Ala Gln Arg Met Lys Leu
   385                 390                 395                 400
   ccg aac ggt gct cgc gtt gaa tat gaa atg cag tgg ggt cac att ggt      1248
   Pro Asn Gly Ala Arg Val Glu Tyr Glu Met Gln Trp Gly His Ile Gly
                   405                 410                 415
   tgg tcc gtt cct gcc gcc ttc ggt tat gcc gtc ggt gct ccg gaa cgt      1296
   Trp Ser Val Pro Ala Ala Phe Gly Tyr Ala Val Gly Ala Pro Glu Arg
               420                 425                 430
   cgc aac atc ctc atg gtt ggt gat ggt tcc ttc cag ctg acg gct cag      1344
   Arg Asn Ile Leu Met Val Gly Asp Gly Ser Phe Gln Leu Thr Ala Gln
           435                 440                 445
   gaa gtt gct cag atg gtt cgc ctg aaa ctg ccg gtt atc atc ttc ttg      1392
   Glu Val Ala Gln Met Val Arg Leu Lys Leu Pro Val Ile Ile Phe Leu
       450                 455                 460
   atc aat aac tat ggt tac acc atc gaa gtt atg atc cat gat ggt ccg      1440
   Ile Asn Asn Tyr Gly Tyr Thr Ile Glu Val Met Ile His Asp Gly Pro
   465                 470                 475                 480
   tac aac aac atc aag aac tgg gat tat gcc ggt ctg atg gaa gtg ttc      1488
   Tyr Asn Asn Ile Lys Asn Trp Asp Tyr Ala Gly Leu Met Glu Val Phe
                   485                 490                 495
   aac ggt aac ggt ggt tat gac agc ggt gct gct aaa ggc ctg aag gct      1536
   Asn Gly Asn Gly Gly Tyr Asp Ser Gly Ala Ala Lys Gly Leu Lys Ala
               500                 505                 510
   aaa acc ggt ggc gaa ctg gca gaa gct atc aag gtt gct ctg gca aac      1584
   Lys Thr Gly Gly Glu Leu Ala Glu Ala Ile Lys Val Ala Leu Ala Asn
           515                 520                 525
   acc gac ggc cca acc ctg atc gaa tgc ttc atc ggt cgt gaa gac tgc      1632
   Thr Asp Gly Pro Thr Leu Ile Glu Cys Phe Ile Gly Arg Glu Asp Cys
       530                 535                 540
   act gaa gaa ttg gtc aaa tgg ggt aag cgc gtt gct gcc gcc aac agc      1680
   Thr Glu Glu Leu Val Lys Trp Gly Lys Arg Val Ala Ala Ala Asn Ser
   545                 550                 555                 560
   cgt aag cct gtt aac aag ctc ctc tag                                  1707
   Arg Lys Pro Val Asn Lys Leu Leu
                   565
<210> 2
   <211> 568
   <212> PRT
   <213> Zymomonas mobilis
<400> 2
   Met Ser Tyr Thr Val Gly Thr Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Asn Leu Leu Leu Asn Lys Asn Met Glu Gln Val Tyr Cys Cys
           35                  40                  45
   Asn Glu Leu Asn Cys Gly Phe Ser Ala Glu Gly Tyr Ala Arg Ala Lys
       50                  55                  60
   Gly Ala Ala Ala Ala Val Val Thr Tyr Ser Val Gly Ala Leu Ser Ala
   65                  70                  75                  80
   Phe Asp Ala Ile Gly Gly Ala Tyr Ala Glu Asn Leu Pro Val Ile Leu
                   85                  90                  95
   Ile Ser Gly Ala Pro Asn Asn Asn Asp His Ala Ala Gly His Val Leu
               100                 105                 110
   His His Ala Leu Gly Lys Thr Asp Tyr His Tyr Gln Leu Glu Met Ala
           115                 120                 125
   Lys Asn Ile Thr Ala Ala Ala Glu Ala Ile Tyr Thr Pro Glu Glu Ala
       130                 135                 140
   Pro Ala Lys Ile Asp His Val Ile Lys Thr Ala Leu Arg Glu Lys Lys
   145                 150                 155                 160
   Pro Val Tyr Leu Glu Ile Ala Cys Asn Ile Ala Ser Met Pro Cys Ala
                   165                 170                 175
   Ala Pro Gly Pro Ala Ser Ala Leu Phe Asn Asp Glu Ala Ser Asp Glu
               180                 185                 190
   Ala Ser Leu Asn Ala Ala Val Asp Glu Thr Leu Lys Phe Ile Ala Asn
           195                 200                 205
   Arg Asp Lys Val Ala Val Leu Val Gly Ser Lys Leu Arg Ala Ala Gly
       210                 215                 220
   Ala Glu Glu Ala Ala Val Lys Phe Thr Asp Ala Leu Gly Gly Ala Val
   225                 230                 235                 240
   Ala Thr Met Ala Ala Ala Lys Ser Phe Phe Pro Glu Glu Asn Ala Asn
                   245                 250                 255
   Tyr Ile Gly Thr Ser Trp Gly Glu Val Ser Tyr Pro Gly Val Glu Lys
               260                 265                 270
   Thr Met Lys Glu Ala Asp Ala Val Ile Ala Leu Ala Pro Val Phe Asn
           275                 280                 285
   Asp Tyr Ser Thr Thr Gly Trp Thr Asp Ile Pro Asp Pro Lys Lys Leu
       290                 295                 300
   Val Leu Ala Glu Pro Arg Ser Val Val Val Asn Gly Ile Arg Phe Pro
   305                 310                 315                 320
   Ser Val His Leu Lys Asp Tyr Leu Thr Arg Leu Ala Gln Lys Val Ser
                   325                 330                 335
   Lys Lys Thr Gly Ser Leu Asp Phe Phe Lys Ser Leu Asn Ala Gly Glu
               340                 345                 350
   Leu Lys Lys Ala Ala Pro Ala Asp Pro Ser Ala Pro Leu Val Asn Ala
           355                 360                 365
   Glu Ile Ala Arg Gln Val Glu Ala Leu Leu Thr Pro Asn Thr Thr Val
       370                 375                 380
   Ile Ala Glu Thr Gly Asp Ser Trp Phe Asn Ala Gln Arg Met Lys Leu
   385                 390                 395                 400
   Pro Asn Gly Ala Arg Val Glu Tyr Glu Met Gln Trp Gly His Ile Gly
                   405                 410                 415
   Trp Ser Val Pro Ala Ala Phe Gly Tyr Ala Val Gly Ala Pro Glu Arg
               420                 425                 430
   Arg Asn Ile Leu Met Val Gly Asp Gly Ser Phe Gln Leu Thr Ala Gln
           435                 440                 445
   Glu Val Ala Gln Met Val Arg Leu Lys Leu Pro Val Ile Ile Phe Leu
       450                 455                 460
   Ile Asn Asn Tyr Gly Tyr Thr Ile Glu Val Met Ile His Asp Gly Pro
   465                 470                 475                 480
   Tyr Asn Asn Ile Lys Asn Trp Asp Tyr Ala Gly Leu Met Glu Val Phe
                   485                 490                 495
   Asn Gly Asn Gly Gly Tyr Asp Ser Gly Ala Ala Lys Gly Leu Lys Ala
               500                 505                 510
   Lys Thr Gly Gly Glu Leu Ala Glu Ala Ile Lys Val Ala Leu Ala Asn
           515                 520                 525
   Thr Asp Gly Pro Thr Leu Ile Glu Cys Phe Ile Gly Arg Glu Asp Cys
   530 535 540
   Thr Glu Glu Leu Val Lys Trp Gly Lys Arg Val Ala Ala Ala Asn Ser
   545 550 555 560
   Arg Lys Pro Val Asn Lys Leu Leu
   565

## Beschreibung

Die Erfindung bezieht sich auf ein Verfahren zur Herstellung von Acyloinen durch enzymatische Umwandlung von alpha-Ketocarbonsäuren und/oder Aldehyden in Gegenwart von Pyruvat-decarboxylase (PDC) sowie eine dafür geeignete neue PDC und deren Herstellung.

Acyloine bzw. alpha-Hydroxyketone sind Verbindungen mit einem optisch aktiven C-Atom, die in der Synthese von komplexeren Verbindungen eine erhebliche Rolle spielen, wie insbesondere das(R)-(-)-Phenylacetylcarbinol (PAC), das für die Produktion von Ephedrin von großem wirtschaftlichem Interessse ist. Hierfür wird das R-Enantiomere benötigt, das durch fermentative Umwandlung von Pyruvat in Gegenwart von Benzaldehyd mittels Saccharomyces cerevisiae gebildet wird (DE-PS 548 459 von 1932).

Bei dieser Synthese von PAC mittels Hefezellen werden auf Grund der Mehrzahl der in der Hefe vorhandenen Enzymen zahlreiche Nebenprodukte gebildet, und das Zellwachstum wird durch die Anwesenheit von Benzaldehyd inhibiert.

Auch die aus der Hefe isolierte Pyruvat-decarboxylase (PDC) führt bei dieser Umsetzung zu erheblichen Anteilen des zu PAC isomeren 2-Hydroxypropiophenons.

Die thiamindiphosphat- and Mg⁺⁺-abhängige PDC (E.C. 4.1.1.1) ist weit verbreitet and wird in vielen Pflanzen, Hefen und anderen Pilzen sowie in einigen Bakterien gefunden. Sie katalysiert die nicht-oxidative Decarboxylierung von Pyruvat zu Acetaldehyd and als Nebenreaktion erfolgt eine Acyloinkondensation unter Bildung von alpha-Hydroxyketonen.

Eine solche enzymatische Umsetzung erfolgt auch ausgehend von einem Aldehyd an Stelle von alpha-Ketocarbonsäuren, und an der Kondensationsreaktion kann auch der durch die Decarboxylierung gebildete Aldehyd als "Cosubstrat" unter Bildung von Homoacyloinen R-CHOH-CO-R' mit R = R' teilnehmen.

In WO 96/37620 wird die Herstellung von Acyloinen aus Acetaldehyd und Benzaldehyd durch Katalyse mit einer gentechnisch veränderten Pyruvatdecarboxylase aus Zymomonas mobilis bechrieben. Es werden insbesondere soche Enzyme als geeignet beschrieben, bei denen dar Tryptophanrest in Position 392 durch einen sterisch kleinereren Rest wie Alanin, Glycin, Phenylalanin, Leucin, Isoleucin Arginin, Histidin, Serin oder Threonin ersetzt ist. Es wird jedoch beschrieben, daß Acetaldehyd alle diese Enzyme inaktiviert.

Iwan et al., J. Molecular Catalysis B: Enzymatic Vol. II, 22. January 2001, S. 387 - 396 beschreiben Mutanten der Pyruvatdecarboxylase (PDC) aus Zymomonas mobilis, welche eine im Vergleich zum Wildtyp höhere Toleranz gegenüber Acetaldehyd aufweisen. Diese werden dadurch erhalten, dass an Position 392 ein Trp-Rest durch Ile oder Met ausgetauscht wird.

WO 99/09195 beschreibt ein Verfahren zur Herstellung von Phenylacetylcarbinol aus Acetaldehyd und Benzaldehyden, wobei als Katalysator eine PDC aus Zymomanas mobilis eingesetzt werden kann, die an Position 392 eine gegenüber dem wildtyp (= Trp) veränderte Aminosäure, bevorzugt Ala, Ile, Met trägt.

Es bestand daher die Aufgabe neue Pyruvatdecarboxylasen bereitzustellen, die die o.g. Nachteile gar nicht oder in wesentlich geringerem Ausmaß aufweisen, ohne daß jedoch die Synthesekapazität der neuen Enzyme reduziert ist.

Gegenstand der Erfindung ist eine Pyruvatdecarboxylase, die gegenüber der PDC aus Zymomonas mobilis eine Aminosäuresubstitution an der Position 553 trägt, wobei das Lysin an dieser Position ausgetauscht ist durch eine Aminoäure ähnlicher Raumerfüllung und/oder Ladung, insbesondere durch die Aminosäure Lysin oder Alanin.

Diese erfindungsgemäße Enzyme (PDC) können ausgehend von der bekannten PDC-Gensequenz aus Zymomonas mobilis durch dem Fachmann bekannte gentechnische Methoden wie site directed mutagenesis hergestellt werden. Hierzu wird ausdrücklich Bezug genommen auf den experimentellen Teil von WO 96/37620, in dem die gentechnische Konstruktion, Expression und Aufreinigung einer PDC Mutante aus Zymomonas mobilis beschrieben ist. Auf Seite 14 der WO 96/37620 ist die für die PDC codierende Nukleinsäuresequenz aus Zymomonas mobilis in 5'-3' Richtung dargestellt, wobei die ersten Nukleotide Nr. 1 bis 3 (ATG) das Startcodon (Methionin, Aminosäureposition +1) für die Translation darstellen und die Nukleotide 1705-1707 (TAG) das Stoppcodon bilden. Aus dieser Nukleotidsequenz ist durch Übersetzung gemäß des genetischen Codes die Aminosäuresequenz der PDC aus Zymomonas mobilis erhältlich.

Eine bevorzugte Ausführungsform der Erfindung betrifft eine PDC bei der das Lysin (K) an Position 553 durch Arginin (R) ausgetauscht ist (R553).

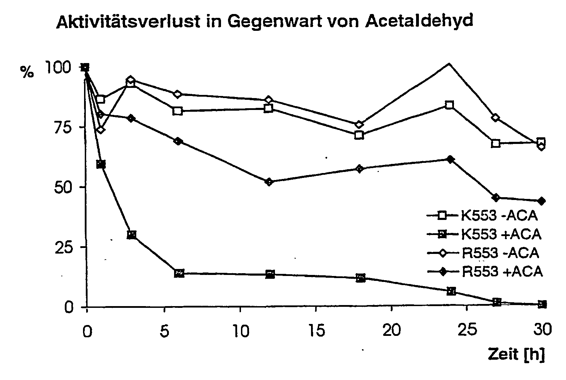

Dieses Enzym weist im Vergleich zum Ausgangsmolekül (K553) eine bessere Stabilität gegenüber Acetaldehyd auf (Fig 1), d.h. der Aktivitätsverlust von R553 ist in Gegenwart von Acetaldehyd (+ACA) wesentlich geringer als bei K553.

Ein weiterer Vorteil dieses Enzyms ist eine deutlich höhere Syntheseleistung (meßbar als Raum/Zeit-Ausbeute) in einem fed batch Experiment im Vergleich zu K553.

Ein weiterer Gegenstand der Erfindung ist ein Verfahen zur Herstellung von enantiomerenreinen Phenylacetylcarbinolen der allgemeinen Formel (I) 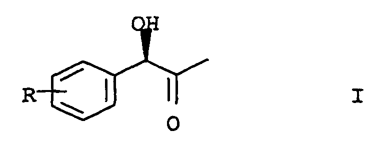 wobei R für H, F, Cl oder Br steht,
aus Acetaldehyd oder Pyruvat und Benzaldehyden der allgemeinen Formel (II) 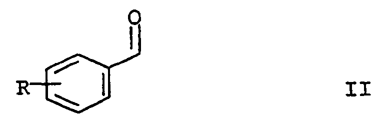 in Gegenwart der erfindungsgemäßen Pyruvatdecarboxylase.

Besonders geeignete Ausführungsformen für dieses erfindungsgemäße Verfahren sind solche, bei denen im Verlauf der Biotransformation Acetaldehyd oder Pyruvat in einer solchen Weise kontinuierlich oder diskontinuierlich nachdosiert wird, daß die Konzentration von Acetaldehyd bzw. Pyruvat im Reaktionsmedium zwischen 20 und 50 mMol/l beträgt.

Hinsichtlich weiterer Einzelheiten dieses Verfahrens wird auf die WO 99/9195 verwiesen, auf die hiermit ausdrücklich Bezug genommen wird. Die Verfahrensführung für das erfindungsgemäße Verfahren ist im wesentlichen identisch mit dem in WO 99/9195 beschriebenen Verfahren mit Ausnahme des eingesetzten Katalysators (Pyruvatdecarboxylase).

### Beispiel 1

### Inaktivierung der PDC durch Acetaldehyd

Die PDC 553R wurde unter den folgenden Bedingungen mit Acetaldehyd behandelt:
30 mM Acetaldehyd
50 mM Morpholinethansulfonsäure
20 mM MgCl2
20 µg zellfreier Proteinrohextrakt
in 100 µl H2O gelöst, pH 7,0

Die Inkubation wurde bei 4°C durchgeführt um eine Reduktion der Acetaldehyd Konzentration durch die enzymkatalysierte Acetoin-Bildung zu unterbinden.

In Fig. 1 ist der Aktivitätsverlust des erfindungsgemäßen Proteins R553 im Vergleich zu dem Ausgangsmolekül K553 in Gegenwart von Acetaldehyd (+ACA) dargestellt. In Abwesenheit von Acetaldehyd (-ACA) sind beide Enzyme etwa gleich stabil über den beobachteten Zeitraum von 30 Stunden.

## Patentansprüche

1. Polypeptid mit Pyruvatdecarboxylase-Aktivität, das sich von der Pyruvatdecarboxylase aus Zymomonas mobilis mit der in SE-QID NO: 2 dargestellten Polynukleotidsequenz durch eine Aminosäuresubstitution in der Position 553 ableitet.

2. Polypeptid nach Anspruch 1, wobei die Aminosäureposition 553 ein Alanin oder Arginin darstellt.

3. Verfahren zur Herstellung von enantiomerenreinen Phenylacetylcarbinolen der allgemeinen Formel (I) 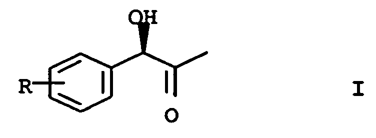 wobei R für H, F, Cl oder Br steht,
aus Acetaldehyd oder Pyruvat und Benzaldehyden der allgemeinen Formel (II) 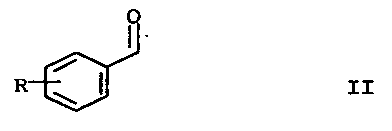 in Gegenwart von Pyruvatdecarboxylase, **dadurch gekennzeichnet, daß** die Pyruvatdecarboxylase ein Polypeptid nach Anspruch 1 oder 2 ist.

4. Verfahren nach Anspruch 3, **dadurch gekennzeichnet, daß** im Verlauf der Biotransformation Acetaldehyd oder Pyruvat in einer solchen Weise kontinuierlich oder diskontinuierlich nachdosiert wird, daß die Konzentration von Acetaldehyd oder Pyruvat im Reaktionsmedium zwischen 20 und 50 mmol/l beträgt.

5. Verfahren nach Anspruch 3, **dadurch gekennzeichnet, daß** ein Benzaldehyd (II) mit R=H eingesetzt wird.

## Claims

1. A polypeptide, possessing pyruvate decarboxylase activity, which is derived from Zymomonas mobilis pyruvate decarboxylase having the polynucleotide sequence shown in SEQ ID NO: 2 by an amino acid substitution at position 553.

2. A polypeptide as claimed in claim 1, wherein the amino acid position 553 constitutes an alanine or arginine.

3. A process for preparing enantiomerically pure phenylacetylcarbinols of the formula (I) 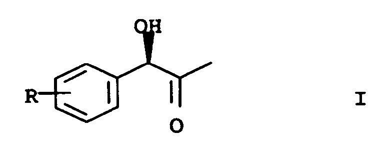 where R is H, F, Cl or Br,
from acetaldehyde or pyruvate and benzaldehydes of the formula (II) 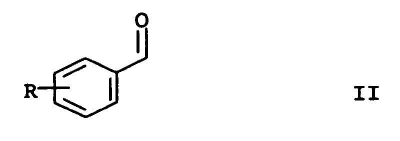 in the presence of pyruvate decarboxylase, wherein the pyruvate decarboxylase is a polypeptide as claimed in claim 1 or 2.

4. A process as claimed in claim 3, wherein acetaldehyde or pyruvate is subsequently metered in continuously or discontinuously, during the course of the biotransformation, such that the concentration of acetaldehyde or pyruvate in the reaction medium is between 20 and 50 mmol/l.

5. A process as claimed in claim 3, wherein a benzaldehyde (II) is employed in which R=H.

## Revendications

1. Polypeptide à activité de pyruvate-décarboxylase, qui dérive de la pyruvate-décarboxylase de Zymomonas mobilis présentant la séquence polynucléotidique représentée dans SEQ ID NO : 2 par une substitution d'acide, aminé dans la position 553.

2. Polypeptide suivant la revendication 1, dans lequel la position d'acide aminé 553 représente une alanine ou arginine.

3. Procédé de préparation de phénylacétylcarbinols exempts d'énantiomères de la formule générale (I) : 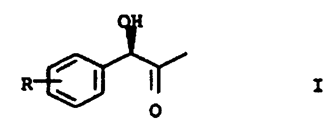 où R représente H, F, Cl ou Br,
à partir d'acétaldéhyde ou de pyruvate et de benzaldéhydes de la formule générale (II) : 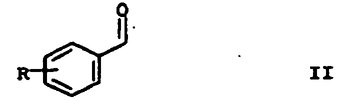 en présence de pyruvate-décarboxylase, **caractérisé en ce que** la pyruvate-décarboxylase est un polypeptide suivant la revendication 1 ou 2.

4. Procédé suivant la revendication 3, **caractérisé en ce que**, au cours de la biotransformation, l'acétaldéhyde ou le pyruvate est dosé ultérieurement de manière continue ou discontinue d'une façon telle que la concentration en acétaldéhyde ou pyruvate dans le milieu réactionnel soit comprise entre 20 et 50 mmoles/litre.

5. Procédé suivant la revendication 3, **caractérisé en ce qu'**on met en oeuvre un benzaldéhyde (II) où R=H.
